# EUROPEAN PATENT APPLICATION

(11) **EP 3 524 667 A1**
(43) Date of publication of application: **14.08.2019**
(21) Application number: 17861674.4
(22) Date of filing: 06.09.2017
(51) Int. Cl.: C12M 1/00, C12M 1/26

(54) **CELL TRANSFER APPARATUS**

(30) Priority: 18.10.2016 JP 2016204575
(71) Applicant: Yamaha Hatsudoki Kabushiki Kaisha, Shizuoka-ken 438-8501 (JP)
(72) Inventor: ITO, Saburo, Iwata-shi Shizuoka 438-8501 (JP)
(74) Representative: Müller-Boré & Partner Patentanwälte PartG mbB
(86) International application number: PCT/JP2017/032059
(87) International publication number: WO 2018/074086

(57) **Abstract**

A cell transfer apparatus includes a controller that controls generation of a suction force and a discharge force at a plurality of heads and controls movement of a head unit. The controller executes a process for specifying a first specimen head to be used for transferring the first specimen and a second specimen head to be used for transferring the second specimen, a process for specifying a first specimen well to be used for receiving the first specimen and a second specimen well to be used for receiving the second specimen, a process for sequentially sucking the cell of the first specimen from the first dish by the first tip attached to the first specimen head and then the cell of the second specimen from the second dish by the second tip attached to the second specimen head, and a process for discharging the cell of the first specimen from the first tip to the first specimen well and the cell of the second specimen from the second tip to the second specimen well.

## Description

### Technical Field

The present invention relates to a cell transfer apparatus that transfers cells from a dish in which the cells are held to a plate having a well for receiving the cells.

### Background Art

For example, in a use for medical and biological studies, a single cell or a cell aggregate obtained by three-dimensionally clumping cells (hereinafter, they are simply referred to herein as cells) are accommodated in wells arranged in a matrix pattern on a microplate in some cases to be subject to processing such as observation, checking for medicinal effect, inspection, or culture. The cells to be accommodated in the wells are sorted on the dish having recessed portions capable of accommodating the cells. Prior to the sorting, a cell culture solution containing a lot of cells is dispersed on the dish, and the cells are held in the recessed portions. Thereafter, images of the dish in which the cells are held are captured, and the cells are classified into usable cells, and unusable cells and foreign substances by an image processing technique. After a while, the usable cells are sucked from the recessed portions by a suction tip, and the sucked cells are discharged onto the wells of the microplate (for example, see Patent Literature 1).

Various kinds of processes on the microplate are executed on a plurality of types of cells in some cases. An example of this case is that cells taken from a plurality of specimens are caused to react with the same compound. In such a case, for example, a conventional cell transfer apparatus sequentially performs an operation for sucking a first cell taken from a first specimen from a first dish where the first cell is dispersed through a tip and discharging the first cell to a well of a microplate, and then sucking a second cell taken from a second specimen from a second dish where the second cell is dispersed through a tip and discharging the second cell to another well on the same microplate.

Herein, the tip that has entered a cell culture solution in the first dish for sucking the first cell cannot be used for sucking the second cell. The tip that has been used for sucking the first cell in the first dish internally accommodates the first cell itself or its piece, or the first cell or the piece adheres to a surface of that tip in some cases. Use of such a tip for sucking the second cell might cause intrusion of the first cell to the well where the second cell is to be held. Further, in the suction of the second cell from the second dish, the first cell adhering to the tip might intrude into the second dish and might be mixed in the second dish which is to accommodate only the second cell. In the above operation, therefore, a replacing operation for the tip is necessary between the suction of the first cell and the suction of the second cell. A wider variety of cells to be processed simultaneously bring about a larger number of the replacing operations and a larger number of tips to be disposed of. Further, every replacing operation for the tip requires operation for taking a cell from a culture container and dispersing the cell to a dish. Thus, such a replacing operation takes a lot of time.

### Citation List

### Patent Literature

Patent Literature 1: WO2015/087371A1

### Summary of Invention

It is an object of the present invention to provide a cell transfer apparatus, which transfers cells to a microplate having wells for receiving the cells from a dish in which the cells are held, can transfer a plurality of types of cells to the microplate efficiently and can reduce the number of tips to be disposed of.

One aspect of the present invention for achieving the above object provides a cell transfer apparatus including a dish group including a first dish where a cell of a first specimen is held and a second dish where a cell of a second specimen is held, the first dish and the second dish each having a plurality of holding portions that holds cells to be transferred, a microplate having a plurality of wells that receives the cells, a head unit that is movable between the dish group and the microplate, the head unit including a plurality of heads and tips attached to the heads, respectively, a suction force and a discharge force being generated at the plurality of heads, the tips being configured to suck and discharge the cells, and a controller that controls the generation of the suction force and the discharge force at the plurality of heads and controls movement of the head unit.

The controller executes a process for specifying at least some of the plurality of heads as a first specimen head to be used for transferring the first specimen and as a second specimen head to be used for transferring the second specimen, a process for specifying some of the plurality of wells as a first specimen well to be used for receiving the first specimen and as a second specimen well to be used for receiving the second specimen, a process for sequentially sucking the cell of the first specimen from the first dish by the first tip attached to the first specimen head and then the cell of the second specimen from the second dish by the second tip attached to the second specimen head, and a process for discharging the cell of the first specimen from the first tip to the first specimen well and the cell of the second specimen from the second tip to the second specimen well.

### Brief Description of Drawings

FIG. 1 is a schematic diagram illustrating a configuration of a cell transfer apparatus according to an embodiment of the present invention.
FIG. 2 is a top view illustrating a dish group in a sorting container to be used in the cell transfer apparatus.
FIG. 3 is a cross-sectional view taken along line III-III in FIG. 2.
FIG. 4 is a top view illustrating a microplate to be used in the cell transfer apparatus.
FIG. 5 is a diagram schematically illustrating a mode of discharging cells from a plurality of tips to the microplate.
FIG. 6 is a schematic diagram illustrating a method for discharging cells according to a comparative example.
FIG. 7 is a top view illustrating a situation where the cells are supported in the microplate, in a case where the discharging method according to the comparative example is employed.
FIG. 8 is a schematic diagram illustrating a method for discharging cells according to a first embodiment.
FIG. 9 is a top view illustrating a situation where the cells are supported in the microplate, in a case where the discharging method according to the first embodiment is employed.
FIG. 10 is a schematic diagram illustrating a method for discharging cells according to a second embodiment.
FIG. 11 is a top view illustrating a situation where the cells are supported in the microplate, in a case where the discharging method according to the second embodiment is employed.
FIG. 12 is a block diagram illustrating an electrical configuration of the cell transfer apparatus.
FIG. 13 is a flowchart illustrating an operation of the cell transfer apparatus.

### Description of Embodiments

Embodiments of the present invention will be described in detail below with reference to the drawings. Subjects to be transferred in the present invention are cells derived from a biological object, particularly, a cell aggregate (spheroid). The cell aggregate derived from a biological object is produced by clumping several cells to hundreds of thousands of cells. For this reason, the cell aggregate varies in size. The cell aggregate produced by living cells has an approximately spherical shape. However, the alternation or death of some cells producing the cell aggregate makes the cell aggregate irregular in shape and density in some cases. In tests for biotechnology and medical technology, a cell transfer apparatus picks up usable cell aggregates from a plurality of cell aggregates having various shapes supported by a dish on a sorting stage and transfers the picked-up cell aggregates to a microplate. In the microplate, the cell aggregates undergo various processes including observation, checking for medicinal effect, inspection, and culture. In the following description, the above-described cell aggregates are simply expressed as cells C.

### [Entire Configuration of Cell Transfer Apparatus]

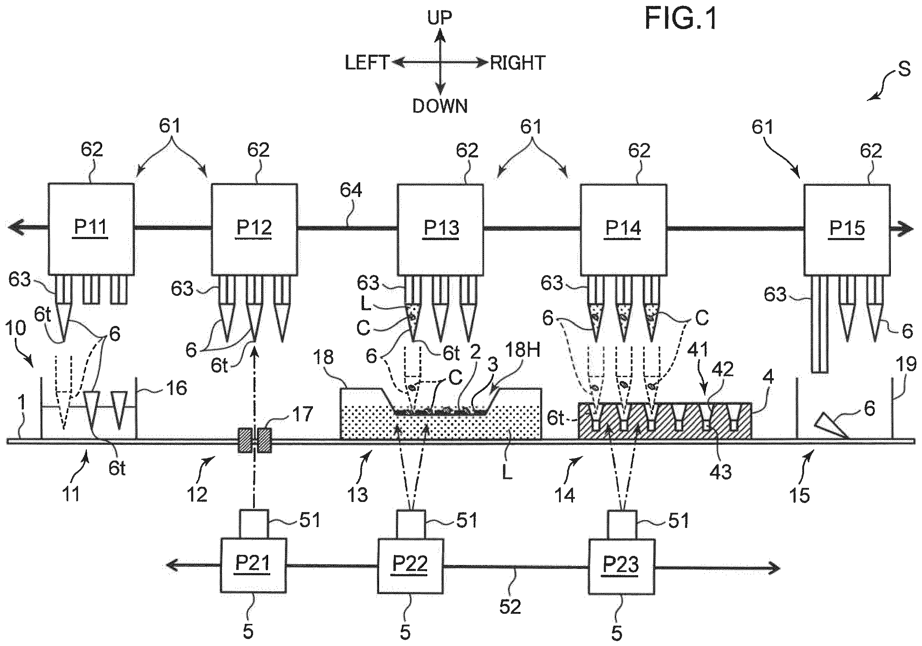

FIG. 1 is a diagram schematically illustrating an entire configuration of a cell transfer apparatus S. The cell transfer apparatus S includes a base 1 (one example of the above-described sorting stage) having a horizontal placing surface (upper surface), a cell transfer line 10 mounted to an upper surface of the base 1, a camera unit 5 disposed below the base 1, a head units 61 that is disposed above the base 1 and mounted with a tip 6 that sucks and discharges the cells C. Note that FIG. 1 illustrates the plurality of camera units 5 and the plurality of head units 61, but they indicate moving positions P11 to P15 of the camera units 5 and moving positions of P21 to P23 of the head unit 61, respectively, but the cell transfer apparatus S includes single camera unit 5 and single head unit 61. Needless to say, the cell transfer apparatus S may include a plurality of camera units 5, and a plurality of head units 61.

The base 1 has predetermined stiffness and is a rectangular flat board, and a part of or an entire part of the base 1 is made by a transparent material. The base 1 is preferably a glass plate. The base 1 made of the transparent material such as the glass plate enables the camera unit 5 disposed below the base 1 to capture an image of respective operating units of the cell transfer line 10 disposed on the upper surface of the base 1 through the base 1.

The cell transfer line 10 includes a plurality of operating units required for carrying out a series of a cell transfer operation for sucking a cell C from one container through the tip 6, transferring the cell C to another container, and discharging the cells C from the tip 6. The operating units are mounted to the base 1 so as to be lined in a right and left direction. The cell transfer line 10 includes, as the plurality of operating units, a tip stock section 11, a tip calibrating unit 12, a sorting unit 13, a transfer unit 14 and a tip disposal unit 15.

The camera unit 5 includes an image pickup device (unillustrated) such as a CCD (charge-coupled device) image sensor, and a camera lens 51 that forms an optical image on a light receiving surface of the image pickup device. The camera unit 5 is movable in the right and left direction along a guide rail 52 extending in the right and left direction and in parallel with the base 1 below the base 1.

The head unit 61 includes a head main body 62, and a plurality of heads 63 that are held by the head main body 62 and are reciprocatable in an up and down direction with respect to the head main body 62. FIG. 1 illustrates three heads 63 arranged in a line, but the number and the arrangement of the heads 63 are not particularly limited. The head unit 61 is movable in the right and left direction along the guide rail 64 extending in the right and left direction and in parallel with the base 1 above the base 1. Note that, although unillustrated in FIG. 1, the head unit 61 is movable also to a direction perpendicular to a sheet surface of FIG. 1 (a front-rear direction).

The head 63 includes a hollow rod whose lower end is opened. The tip 6 is mounted on a lower end of the head 63. The tip 6 is a tubular member that is tapered toward a tip end and includes a front end opening 6t. A piston mechanism is mounted into the hollow portion of the head 63, and an operation of the piston mechanism applies a suction force and a discharge force to the lower end opening. The head main body 62 contains a power unit of the piston mechanism, an ascending and descending mechanism that moves the head 63 in the up and down direction, and a power unit of the ascending and descending mechanism. The generation of the suction force and the discharge force at the heads 63 generates a suction force and a discharge force also at the front end openings 6t of the tips 6 attached to the heads 63. These forces at the front end openings 6t cause the tips 6 to suck and discharge the cells C through the front end openings 6t.

### [Details of Cell Transfer Line]

The respective operating units of the cell transfer line 10 will be described below. The tip stock section 11 is a portion where a lot of unused tips 6 are stored. A stock container 16 where the tips 6 arranged in a matrix pattern in a standing state are held is disposed in the tip stock section 11. The tips 6 are held in the stock container 16 with their upper end openings facing upward. That is, the tips 6 are held in the stock container 16 so as to be easily attached to lower ends of the heads 63 which move in the up and down direction.

The tip calibrating unit 12 obtains positions (XYZ coordinates) of the front end openings 6t of the tips 6 attached to the heads 63. The tip calibrating unit 12 is provided with an image capture pit 17 with which the camera unit 5 images the tips 6 attached to the heads 63. The tip calibrating unit 12 obtains the XYZ coordinates of the front end openings 6t of the tips 6 based on the image of the tips 6 and focal point position information in the image capture.

The sorting unit 13 sorts the cells C to be transferred. The sorting container 18 is disposed in the sorting unit 13. The sorting container 18 is a container that is a transfer source of the cells C, stores a culture medium L, and holds a dish 2 (a dish group) for cell sorting with the dishes 2 being immersed in the culture medium L. The dish 2, which is a plate that supports the cells C, has a plurality of holding recessed portions 3 (holding portions) on an upper surface of the dish 2. The holding recessed portions 3 can hold the cells C separately.

The culture medium L is not particularly limited as long as it prevents deterioration in properties of the cells C, and thus any culture medium can be selected appropriately based on types of the cells C. Examples of the culture medium L include a base medium, a synthetic medium, an Eagle medium, a Roswell Park Memorial Institute (RPMI) medium, a Fischer's medium, a Ham's medium, a Molecular, Cellular, and Developmental Biology (MCDB) medium, and a serum medium, as well as cell freezing liquids such as glycerol and a cell banker (manufactured by Juji Field Inc.) to be added before cryopreservation, formalin, a reagent for fluorescent stain, an antibody, purified water, and normal saline. For example, in the case where BxPC-3 (human pancreatic adenocarcinoma cells), which is a cell derived from a biological object is used as the cell C, the culture medium L to be used is created by blending 10% of a fetal bovine serum (FBS) in a RPMI-1640 medium and adding antibiotic and a supplement such as sodium pyruvate if necessary.

The sorting container 18 has a cylindrical or square-tubular shape, and includes an upper opening 18H having an orthogonal shape on an upper surface of the sorting container 18. The upper opening 18H is used for putting the cells C into the sorting container 18 and picking up sorted cells C. The dish 2 is disposed below the upper opening 18H. The sorting container 18 and the dishes 2 to be used are made of a transparent resin material or glass. Such a material enables the cells C supported by the dish 2 to be observed through the camera unit 5 disposed below the sorting container 18.

A plurality of cells C that has been dispersed in a cell culture solution are put into the sorting container 18 through a dispensing tip, unillustrated. The dispensing tip sucks the cell culture solution along with the cells C from a tube that stores the cell culture solution containing a lot of the cells C, and internally holds the sucked cell culture solution. Thereafter, the dispensing tip is moved above the sorting container 18, and accesses to the upper surface of the dish 2 through the upper opening 18H. Then, the cells C held in the tip are discharged along with the cell culture solution with a front end opening of the dispensing tip being immersed in the culture medium L of the sorting container 18. The cell transfer apparatus S includes a cell stock section in which the tube is disposed and a dispensing tip stock section that stores a plurality of the dispensing tips, although they are omitted in FIG. 1.

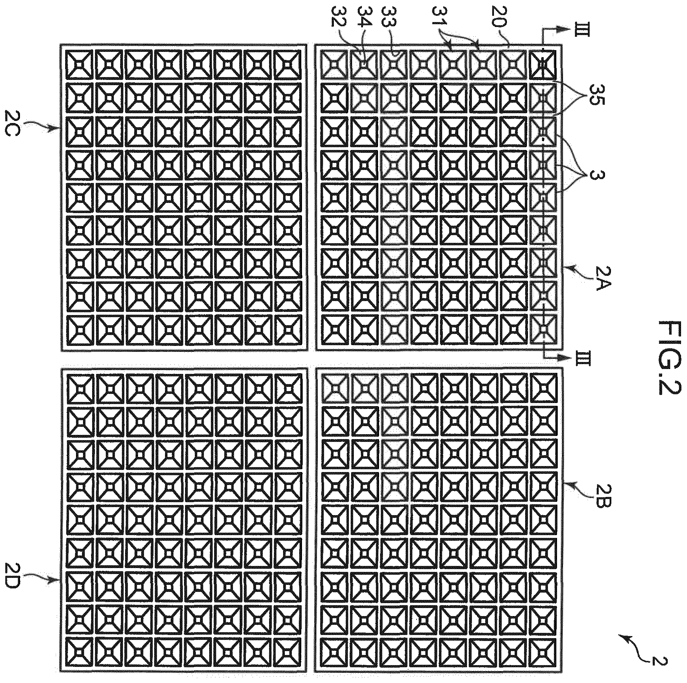

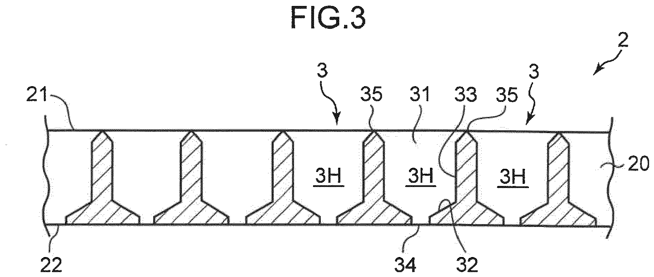

FIG. 2 is a top view of the dish 2, and FIG. 3 is a cross-sectional view taken along line III-III in FIG. 2. The dish 2 illustrated here includes a dish group configured by disposing four square dishes, namely, first, second, third, and fourth dishes 2A, 2B, 2C, and 2D into one big square shape. The first to fourth dishes 2A to 2D each includes a dish main body 20, and a plurality of holding recessed portions 3 formed in the dish main body 20. The holding recessed portions 3 that hold the cells C of micron order on the dish 2 have a minute size, and thus a thin plate is naturally used in the dish main body 20. In this case, since enlargement of the dish size deteriorates flatness of the dish, the dish 2 is formed into a desired size by collecting the first to fourth dishes 2A to 2D with small size.

In the present embodiment, the cells C taken from a first specimen such as a human body or an animal are held by the holding recessed portions 3 of the first dish 2A, and the cells C taken from another second specimen are held by the holding recessed portions 3 of the second dish 2B. In such a manner, each of the first to fourth dishes 2A to 2D is assumed to be allocated to a corresponding specimen. In this case, the dispensing tips discharge the cell culture solutions containing the cells C prepared for the respective specimens onto the first to fourth dishes 2A to 2D allocated to the corresponding specimens, respectively.

The dish main bodies 20 of the first to fourth dishes 2A to 2D each are made of a flat board-shaped member having a predetermined thickness and include an upper surface 21 and a lower surface 22. The plurality of holding recessed portions 3 that holds the cells C to be transferred are disposed on the upper surface 21. The dish 2 is immersed in the culture medium L in the sorting container 18. For details, the dish 2 is held in the sorting container 18 with the upper surface 21 of the dish main body 20 being immersed in the culture medium L in the sorting container 18, whereas with the lower surface 22 being separated from a bottom plate of the sorting container 18 (see FIG. 1).

Each of the holding recessed portions 3 includes an opening 31, a bottom portion 32, a tubular wall surface 33, a hole portion 34, and a boundary portion 35. The present embodiment describes an example where the holding recessed portions 3 having a square shape when viewed from the top are arranged in a matrix pattern. The opening 31 is a square opening disposed on the upper surface 21, and has a size sufficient to allow the front end opening 6t of the tip 6 for sorting to pass therethrough. The bottom portion 32 is disposed near the lower surface 22 inside the dish main body 20. The bottom portion 32 is a tilted surface that gently tilts down toward the center (the center of the square). The tubular wall surface 33 is a wall surface that extends vertically downward the bottom portion 32 from the opening 31. The hole portion 34 is a through hole that vertically pierces between the center of the bottom portion 32 and the lower surface 22. The hole portion 34 has a square shape when viewed from the top, and is concentric with the opening 31. The boundary portion 35 that is disposed on the upper surface 21 is a ridge line that forms an opening edge of each of the holding recessed portions 3 and separates the holding recessed portions 3 from each other. Note that each of the holding recessed portions 3 may have a circular, triangular, pentagonal, or hexagonal shape when viewed from the top, and they may be disposed in the dish main body 20 into a honeycomb, linear, or random pattern. Alternatively, the dish 2 may have only one holding recessed portion 3.

The bottom portions 32 and the tubular wall surfaces 33 of the holding recessed portions 3 define accommodation spaces 3H that accommodates the cells C. Generally, each of the accommodation spaces 3H is designed to accommodate one cell C. Therefore, each of the holding recessed portions 3 is set based on a size of a target cell C. In dispersion of a cell culture solution containing a lot of the cells C into the sorting container 18, however, the plurality of cells C enter one holding recessed portion 3 in some cases. The hole portions 34 are disposed to release small cells other than cells with desired size and foreign substances through the accommodation spaces 3H. Therefore, the hole portions 34 each has a size such that the cells C with desired size fail to pass but small cells other than the cells C with desired size or foreign substances pass through the hole portions 34. As a result, the cells C to be sorted are trapped in the holding recessed portions 3, whereas foreign substances or the like drop from the hole portion 34 onto the bottom plate of the sorting container 18.

The transfer unit 14 transfers the cells C sorted in the sorting unit 13. A microplate 4 is disposed in the transfer unit 14. The microplate 4 is a container that is a transfer destination of the cells C, and includes a plurality of wells 41 that receive the cells C. One of the wells 41 accommodates a necessary number (normally one) of the cells C along with the culture medium L. The microplate 4 to be used is also made of transparent resin material or glass. This material enables the cells C supported by the microplate 4 to be observed through the camera unit 5 disposed below the microplate 4.

As illustrated in FIG. 1, the wells 41 each include a tapered portion 42 and a tubular portion 43 connected with a lower part of the tapered portion 42. The tapered portion 42 has a circular opening on an upper surface of the microplate 4, and has a tapered shape such that a diameter gradually decreases downward from the upper surface. The tubular portion 43 has an inner diameter that is uniform in the up and down direction, and includes a bottom portion on its lower end. Similarly to the example of the dish 2 illustrated in FIG. 2, the plurality of small microplates may, for example, be integrated in a frame member to form one microplate 4.

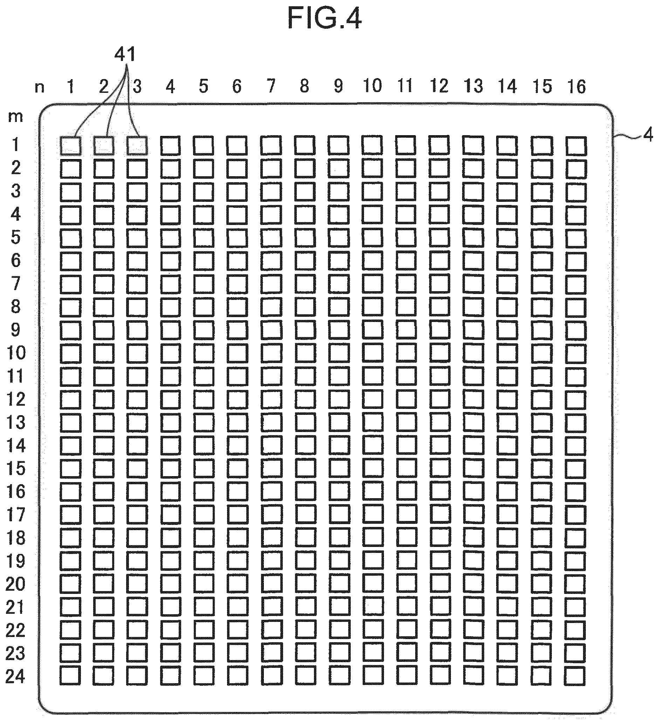

FIG. 4 is a top view illustrating the microplate 4. The wells 41 are arranged in a matrix pattern of m rows ×n columns with a predetermined pitch. A standard plate of 85.48 mm × 127.76 mm is used as the microplate 4 (see "Footprint Dimensions-for Microplates" defined by Society for Laboratory Automation and Screening (SLAS) of American National Standards Institute (ANSI) in 2004). In this case, a general number of the wells 41 is, as illustrated in FIG. 4, obtained as m rows × n columns = 24 rows × 16 columns = 384. The wells 41 are arranged on the base of the microplate 4 in the matrix pattern with the predetermined pitch.

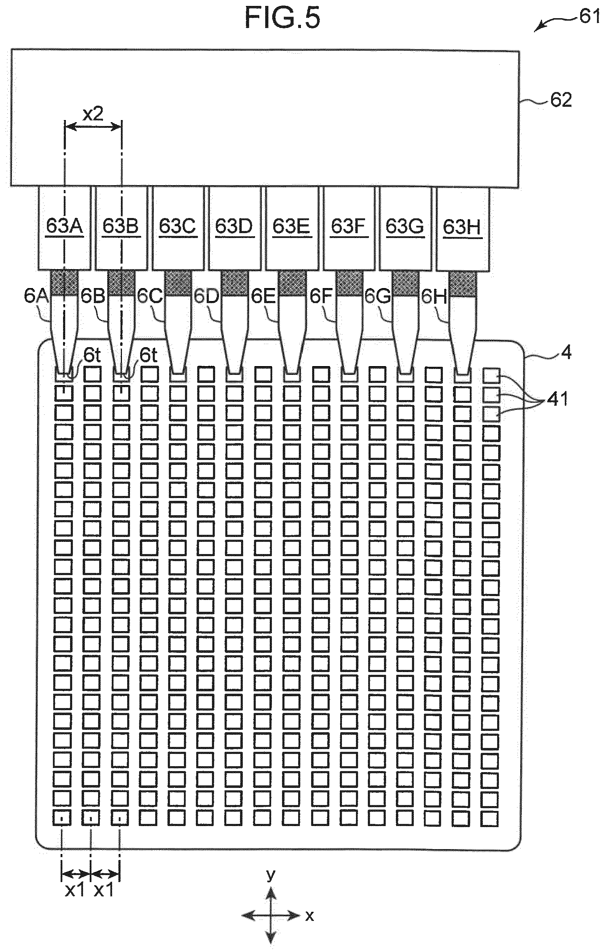

FIG. 5 is a diagram schematically illustrating a mode of discharging cells from the plurality of tips to the microplate, and illustrating a relationship between an arrangement pitch of the tips attached to the heads 63 and the arrangement pitch of the wells 41. Herein, FIG. 5 illustrates the head unit 61 in which the head main body 62 includes eight heads 63A, 63B, 63C, 63D, 63E, 63F, 63G, and 63H that are aligned in a line, and tips 6A, 6B, 6C, 6D, 6E, 6F, 6G, and 6H are attached to the heads 63A to 63H, respectively.

The wells 41 of the microplate 4 are arranged in an x direction (a row direction) with a uniform pitch x1. The tips 6A to 6H (the front end openings 6t) attached to the eight heads 63A to 63H, respectively, are arranged in the x direction with a pitch x2 which is twice the pitch x1. The pitch x2 of the tips 6A to 6H is not limited to twice the pitch x1, and thus may be p multiple (p is an integer of 1 or more) of the pitch x1. In such an arrangement of the heads 63A to 63H (the tips 6A to 6H), when, for example, the front end opening 6t of the tip 6A is aligned with one well 41 as a discharge target, also the tips 6B to 6H are sequentially aligned with alternate wells 41 in the x direction. This alignment enables the cells C to be discharged simultaneously from the tips 6A to 6H and to be put into the wells 41. Such simultaneously discharge can reduce the movement time of the head unit 61 and the number of times of up-and-down movements of the heads 63A to 63H, and thus can shorten a time required for the transfer of the cells C.

Herein, the simultaneously discharge is not necessarily limited to that the cells C are discharged from the tips 6A to 6H at the same timing. That is, assumed "simultaneous discharge" in this specification includes various modes for discharging the cells C from the tips 6A to 6H without a movement of the head unit 61, such as a mode for discharging the cells C from all the tips 6A to 6H at the same timing with the wells 41 being aligned with the tips 6A to 6H as illustrated in FIG. 5, and a mode for discharging the cells from some or all of the tips 6A to 6H at different timings.

The tip disposal unit 15 collects used tips 6 which have completed the sucking and discharging operation from the heads 63. The tip disposal unit 15 includes a tip collecting container 19 that collects the used tips 6. In the disposal, the head unit 61 equipped with the used tips 6 is moved above the opening of the tip collecting container 19, and the tips 6 are removed from the heads 63. This removal operation causes the tips 6 to drop into the tip collecting container 19.

### [Description about Cell Transfer Operation]

A cell transfer operation to be performed by the cell transfer apparatus S will be described with reference to FIG. 1. A basic procedure of the cell transfer operation includes a step (1) of attaching the tip 6 to the head 63, a step (2) of calibrating a position of the front end opening 6t of the tip 6, a step (3) of picking a cell C up from the sorting container 18 (the dish 2), a step (4) of transferring the cell C to the microplate 4, and a step (5) of disposing of the tip 6. For sequential execution of this procedure, the head unit 61 is moved from left to right along the guide rail 64 above the respective operating units of the cell transfer line 10. The camera unit 5 images the tip 6 attached to the head 63 to obtain the position of the front end opening 6t in the step (2), images the dish 2 to select usable cell C before the step (3), and images the microplate 4 to check the transferred cell C after the step (4). The respective steps (1) to (5) will be described below.

In the step (1), the head unit 61 is moved to a tip attachment position P11 above the tip stock section 11. At this time, the head unit 61 is stopped in a position where one of the tips 6 held in the stock container 16 is aligned with one of the heads 63 on a vertical axis. As indicated by a dotted line in FIG. 1, one of the heads 63 moves down, and an upper end portion of the tubular tip 6 is fitted to a lower end of the head 63. Thereafter, the head 63 is caused to move up. The tips 6 are attached also to the other heads 63 similarly.

To execute the step (2), the head unit 61 is moved to a tip calibration position P12 above the tip calibrating unit 12. At this time, the head unit 61 is stopped in a position where one head 63 to which the tip 6 is newly attached is aligned with the image capture pit 17 on a vertical axis. On the other hand, the camera unit 5 is also moved to a tip imaging position P21 just below the image capture pit 17 of the tip calibrating unit 12. The camera unit 5 images the tip 6 located above the image capture pit 17.

The position of the front end opening 6t can be obtained through, for example, a contrast detection method. Specifically, while a focus position is being shifted up from a predetermined position below the front end opening 6t as the image capture starting position in tens of micron by the camera lens 51, the camera unit 5 is caused to sequentially capture images of the tip 6. An imaging end point is a predetermined position that can be defined above the front end opening 6t. An image on which a line estimated as the front end opening 6t appears with highest contrast is selected from the obtained images, and a focus position where the selected image is captured is treated as an in-focus position and a coordinate position of the front end opening 6t is obtained based on a focus distance. This coordinate position is compared with a reference position where the tip 6 is properly attached to the head 63, and a correction value is derived from a difference between the coordinate position and the reference position. This correction value is used as a correction value when the movement of the head unit 61 (the head 63) is controlled. Similar imaging and similar deriving of the correction value are performed also for the other heads 63.

In the step (3), the head unit 61 is moved to a cell suction position P13 above the sorting unit 13. Before execution of the step (3), cell suspensions containing cells C of respective specimens are dispersed onto the first to fourth dishes 2A to 2D in the sorting container 18. Thus, the cells C are supported by the dishes 2A to 2D. Thereafter, the camera unit 5 is moved to a dish imaging position P22 below the sorting unit 13, and images the dishes 2A to 2D where the cells C are supported. Note that, since an angle of view of the camera unit 5 is smaller than a dish size, the imaging operation is performed multiple times. Usable cells C are determined based on these images, and coordinates of the holding recessed portions 3 that support the usable cells C are specified. A suction sequence is set so that a determination is made as to which cell C is sucked by which head 63 (tip 6) and in what order the suction is performed. Further, a discharge sequence is also set as to which head 63 (tip 6) to discharge the cell to which well 41 in the microplate 4.

After the setting the suction sequence, the tip 6 that first performs suction is aligned with the holding recessed portion 3 as a suction target in the dish 2 with reference to the correction value obtained in the step (2), and the heads 63 moves down. When the front end opening 6t of the tip 6 enters the culture medium L in the sorting container 18 and faces the holding recessed portion 3 as a target, a suction force is generated at the head 63. The suction force causes the cell C supported by the holding recessed portion 3 as the target to be sucked into the tip 6. Thereafter, the head 63 is caused to move up. The above-described same operation is performed sequentially on the holding recessed portions 3 associated with subsequent tips 6 according to the suction sequence, and thus the cells C are sucked by the tips 6.

In the step (4), the head unit 61 is moved to a cell discharge position P14 above the transfer unit 14. That is, the head unit 61 is moved above the microplate 4 from the dish 2. The head unit 61 is stopped so that the tips 6 that hold the cells C are aligned vertically with the wells 41 as the discharge targets in the microplate 4. The heads 63 moves down until the front end openings 6t of the tips 6 enter the openings of the wells 41. The discharge forces are generated at the heads 63 to discharge the cells C held in the tips 6 from the front end openings 6t onto the wells 41. As described above with reference to FIG. 5, in the discharge, the plurality or all of the heads 63 move down simultaneously, and the cells C are discharged simultaneously from the tips 6 attached to the heads 63.

In the step (4), the camera unit 5 is also moved to a microplate imaging position P23 below the transfer unit 14. After the discharge of the cells C onto the wells 41 is completed, the camera unit 5 captures an image of the microplate 4 where the cells C are supported. This image can make a situation where the cells C are supported in the microplate 4 obvious. Thereafter, the microplate 4 where the cells C are supported is to be used for the various processes including observation of the cells C, a check for a medicinal effect, inspection, and culture. A typical example of the processes is an experiment in which compounds under test is added to the wells 41 and reactions of the compounds are observed.

In the step (5), the head unit 61 is moved to a tip disposal position P15 above the tip disposal unit 15. The tip collecting container 19 whose upper surface is opened is disposed in the tip disposal unit 15. The head 63 moves down toward the tip collecting container 19, and a tip removal rod (unillustrated) contained in the head 63 moves down. The tip 6 is pressurized by downward movement of the rod and is removed from the head 63. The removed tip 6 drops into the tip collecting container 19. This removal operation is performed according to a contamination degree of the tip 6 after several to ten sucking and discharging operations are performed when the cells C of the same specimen are sucked and discharged. When cells of different specimens are sucked and discharged, the removal operation is performed every time when a specimen is changed.

### [Mode for sucking and Discharging Cells of each Specimen]

The cell transfer apparatus S according to the present embodiment performs the cell transfer operation as described above, and this operation uses a method that enables the number of replacing operations for the tips 6 to be as small as possible when a plurality of specimens are present. For example, cells C taken from a first specimen are supported by the first dish 2A (FIG. 2), and cells C taken from a second specimen are supported by the second dish 2B. Since the tips 6 that have contacted with the culture medium L in the first dish 2A for sucking the cells C of the first specimen are contaminated, the tips 6 cannot allow to contact with the culture medium L in the second dish 2B for sucking cells C of the second specimen. Therefore, the replacing operation for the tips 6 is necessary for the sucking the cells C of the second specimen. Under the limit of such a situation, the cell transfer apparatus S according to the present embodiment can suppress the number of the replacing operations for the tip or eliminate the replacing operations for the tip even when a plurality of types of cells C are transferred.

### <Comparative Example>

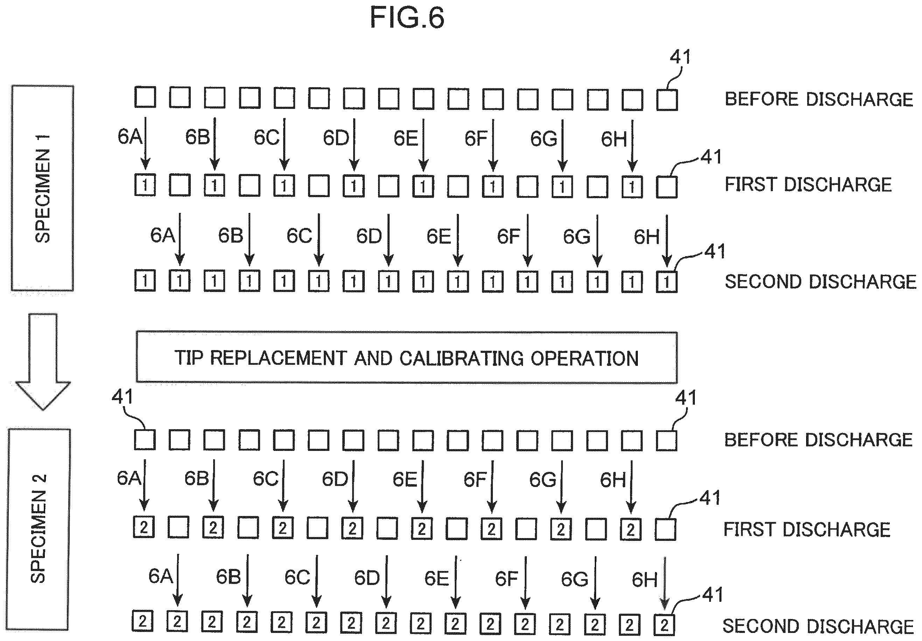

A comparative example of the cell sucking and discharging mode in the case where a plurality of specimens are present will be first described with reference to FIG. 6. Herein, as illustrated in FIG. 5, the head unit 61 includes the eight heads 63A to 63H and the tips 6A to 6H attached to the heads 63A to 63H, respectively. The operation for sucking and discharging the cells C is performed in the head unit 61. Further, the microplate 4 is used that includes the wells 41 of m rows × n columns = 24 rows × 16 columns. The arrangement pitch of the tips 6A to 6H is twice a pitch of the wells 41 in a row direction. In FIG. 6, the eight tips 6A to 6H are indicated by arrows in a simplified manner, and sixteen wells 41 for one row are illustrated.

In the comparative example, in the case where cells C of a specimen 1 belonging to a specific individual and cells C of a specimen 2 belonging to an individual different from the specimen 1 are present, the cells C of the specimen 1 are sucked and discharged and then the cells C of the specimen 2 are sucked and discharged. In such a manner, the suction and the discharge are performed simply in order of specimens.

When the cells C are discharged simultaneously to the sixteen wells 41 for one row by using the eight tips 6A to 6H arranged in one row, the two simultaneous discharging operations are performed for one row. For example, the cells C of the specimen 1 determined to be usable are sucked from the first dish 2A by the eight tips 6A to 6H (the first suction). The head unit 61 is moved to the microplate 4, and the cells C sucked by the tips 6A to 6H are discharged to eight of the sixteen wells 41 in the first discharging operation. In FIG. 6, "1" described in squares representing the wells 41 means that the cells C of the specimen 1 are discharged to the wells 41 (much the same is true on the following description). That is, although the sixteen wells 41 are free before the first discharge, the cells C of the specimen 1 are supported by alternate eight wells 41 in the first discharge.

Thereafter, in the second suction, the cells C of the specimen 1 are sucked from the first dish 2A by the eight tips 6A to 6H. Thereafter, the head unit 61 is moved to the microplate 4, and the second discharging operation is performed so that the cells C are discharged to the residual eight wells 41 which are not subject to the first discharge from the tips 6A to 6H. As a result, the cells C of the specimen 1 are supported by all the sixteen wells 41 for one row. The same operating sequence is performed for necessary rows.

Thereafter, prior to the sucking and discharging operation for the cells C of the specimen 2, the replacing operation for the tips 6 is necessary. The replacing operation includes a step of moving the head unit 61 to the tip disposal unit 15 and removing used tips 6, a step of moving the head unit 61 to the tip stock section 11 and attaching unused tips 6 to the heads 63, and a step of moving the head unit 61 to the tip calibrating unit 12 and obtaining XYZ coordinates of the front end openings 6t of the tips 6 newly attached to the heads 63.

After the above replacing operation, for example, the cells C of the specimen 2 are sucked by the eight tips 6A to 6H from the second dish 2B in the first suction, and the head unit 61 is moved to the microplate 4. The cells C of the specimen 2 sucked by the tips 6A to 6H are then discharged to eight of the sixteen wells 41 in the first discharging operation. In FIG. 6, "2" described in the squares representing the wells 41 means the wells 41 where the cells C of the specimen 2 have been discharged. The cells C of the specimen 2 are supported by alternate eight wells 41 by the first discharge. The second suction and discharge are performed, and thus the cells C of the specimen 2 are supported by all the sixteen wells 41 for one row. The same operating sequence is performed for necessary rows. In a case where another specimen is present, the same operating sequence is repeated.

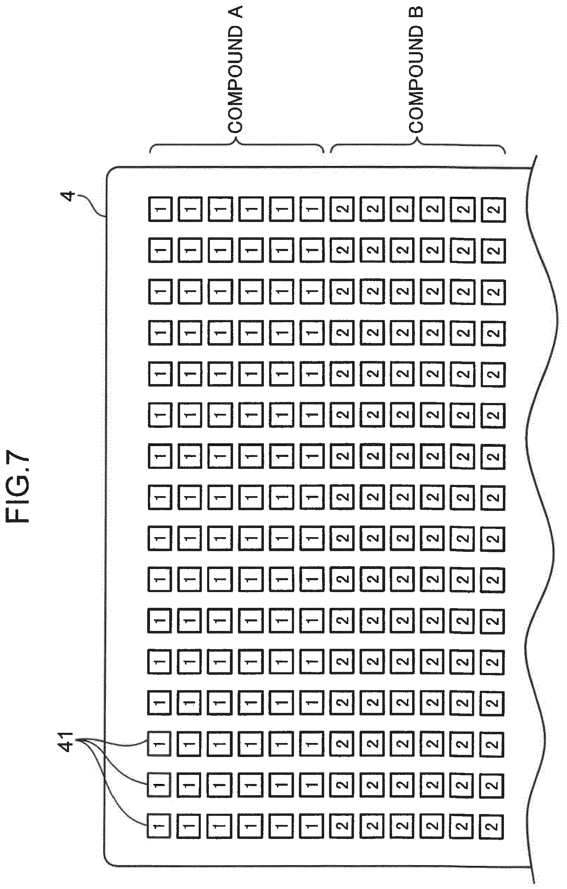

FIG. 7 is a top view illustrating a situation where the cells C are supported in the microplate 4, in a case where the discharging method according to the comparative example is employed. The cells C of the specimen 1 are supported in a group of the wells 41 of 6 rows × 16 columns, and the cells C of the specimen 2 are supported in an adjacent group of the wells 41 of 6 rows × 16 columns. For example, a "compound A" is put into the wells 41 that support the cells C of the specimen 1, and a "compound B" is put into the wells 41 that support the cells C of the specimen 2. After that, the microplate 4 where the cells C are supported is used for an experiment in which sensitivities of the cells C to these compounds are checked.

The above-described method according to the comparative example requires the replacing operation for the tips 6 prior to the sucking and discharging operation to be performed on cells C of every specimen. Since the replacing operation requires a reasonable period of time, the cell transfer operation takes a long period of time. Further, the larger number of specimens (wider variety of cells) to be processed requires a larger number of replacing operations, and thus a great deal of time is required. Further, this situation increases the number of tips 6 to be disposed of, and this is not preferable in view of the cost. In addition, when the cells C of the specimen 2 are dispersed to the dish 2 after the discharge of the cells C of the specimen 1 is completed, a setup operation is required every time when the replacing operation occurs, and thus a great deal of time is consumed.

### <Embodiments>

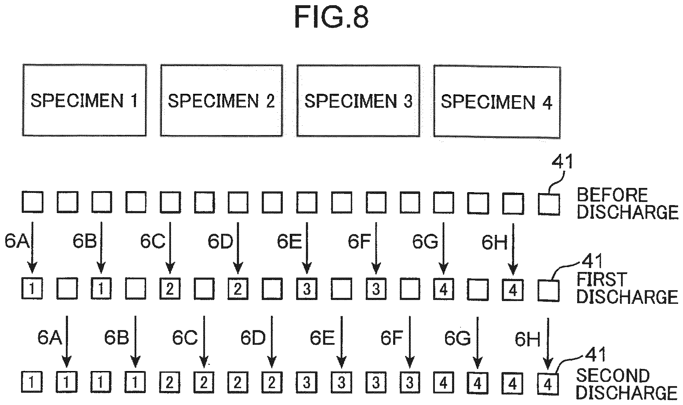

A cell sucking and discharging mode according to a first embodiment in the case where a plurality of specimens are present will be described below with reference to FIG. 8. The head unit 61 including the eight tips 6A to 6H is used, and the microplate 4 including the wells 41 of 24 rows × 16 columns is used. That is, the conditions are equal between the first embodiment and the comparative example. Four specimens 1 to 4 are present, and cells C of the specimens 1 to 4 are supported by the first to fourth dishes 2A to 2D, respectively (FIG. 2).

In the present embodiment, the eight heads 63A to 63H (the tips 6A to 6H) are allocated to the specimens 1 to 4. FIG. 8 illustrates an example where the tips 6A and 6B are allocated to (specified for) the specimen 1 (the first specimen head), the tips 6C and 6D are allocated to the specimen 2 (the second specimen head), the tips 6E and 6F are allocated to the specimen 3, and the tips 6G and 6H are allocated to the specimen 4.

In the first suction, the cells C of the specimen 1 are sucked by the tips 6A and 6B in the first dish 2A, the cells C of the specimen 2 by the tips 6C and 6D in the second dish 2B, the cells C of the specimen 3 by the tips 6E and 6F in the third dish 2C, and the cells C of the specimen 4 by the tips 6G and 6H in the fourth dish 2D. The head unit 61 is moved to the microplate 4, and the cells C sucked by the tips 6A to 6H are discharged to the alternate eight wells 41 in the sixteen wells 41 by the first discharging operation. As illustrated in FIG. 8, each two cells C of the specimens 1 to 4 are supported by the wells 41 by the first discharge.

Thereafter, similarly, each two cells C of the specimens 1 to 4 are sucked from the first to fourth dishes 2A to 2D by the eight tips 6A to 6H in the second suction. Thereafter, the head unit 61 is moved to the microplate 4, and the second discharging operation is performed so that the cells C are discharged to the residual eight wells 41 which are not subject to the first discharge from the tips 6A to 6H. As a result, each four cells C of the specimens 1 to 4 are supported by each four wells 41 in the sixteen wells 41 for one row. The same operating sequence is performed for necessary rows. In such a manner, each of the heads 63 is specified for each of the specimens, and the suction and the discharge are performed only on these specimens by the specified heads 63. This manner can omit the replacing operation for the tips 6 or can reduce the number of the replacing operations for the tips 6 greatly during the cell transfer operation, and thus can shorten the operation time.

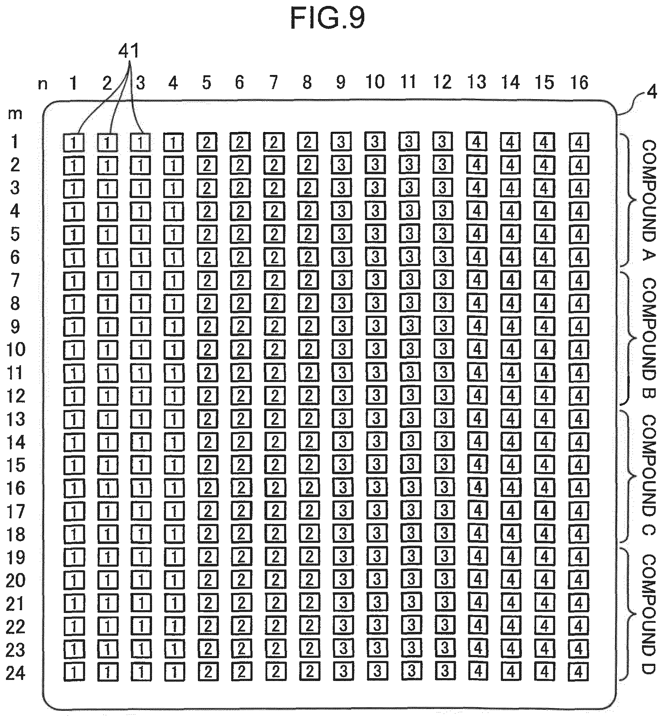

FIG. 9 is a top view illustrating a situation where the cells C are supported in the microplate 4, in a case where the discharging method according to the first embodiment is employed. The cells C of the specimen 1 are supported by the wells 41 of m1 to m24 rows × n1 to n4 columns, and the cells C of the specimen 2 by the wells 41 of m1 to m24 rows × n5 to n8 columns, the cells C of the specimen 3 by the wells 41 of m1 to m24 rows × n9 to n12 columns, and the cells C of the specimen 4 by the wells 41 of m1 to m24 rows x n13 to n16 columns.

For example, as illustrated in FIG. 9, a "compound A" is put into the wells 41 of m1 to m6 rows × n1 to n16 columns, a "compound B" is put into the wells 41 of m7 to m12 rows × n1 to n16 columns, a "compound C" is put into the wells 41 of m13 to m18 rows × n1 to n16 columns, and a "compound D" is put into the wells 41 of m19 to m24 rows × n1 to n16 columns. After that, the microplate 4 where the cells C are supported can be used for an experiment in which sensitivities of the cells C to the compounds A to D are checked.

For example, since the wells 41 for four columns are specified for the specimens 1 to 4, concentrations of the compounds to be added are variable among the respective rows. Specifically, a use method of the wells 41 where compound concentrations are gradually decreased is exemplified for the specimen 1. That is, compounds A to D with highest concentration are put into the wells 41 on the first row, and compounds A to D with lowest concentration are put into the wells 41 on the fourth row. In another exemplified use method of the wells 41, although wells 41 on six rows are specified for each of the compounds A to D, for example, concentration distribution is completely equal among the respective rows (six combinations of the compounds with the equal concentration are formed), and a number of experiment samples increases.

In the above-described operation for discharging the cells from the tips 6A to 6H to the wells 41 in the microplate 4, when the compounds A to D are put into the wells 41 after the cell discharge, the replacement of the tips 6A to 6H is unnecessary. On the other hand, when the compounds A to D are put into the wells 41 in advance, the tips 6A to 6H need to be replaced during the plurality of discharging operations. In the latter case, in the first discharging operation, the tips 6A to 6H come in contact with the compounds A to D in the wells 41. In this case, when the second sucking operation is executed without replacing the tips 6A to 6H, the culture medium L and the specimens 1 to 4 in the first to fourth dishes 2A to 2D are affected by the compounds A to D.

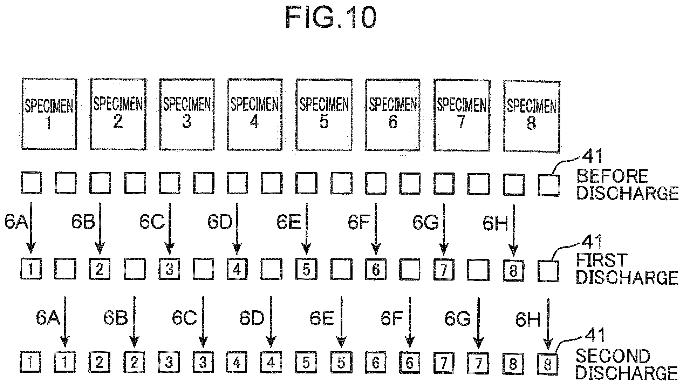

FIG. 10 is a pattern diagram illustrating a cell sucking and discharging method according to the second embodiment. In this example, eight specimens 1 to 8 are present, the tip 6A is specified for the specimen 1, the tip 6B for the specimen 2, the tip 6C for the specimen 3, the tip 6D for the specimen 4, the tip 6E for the specimen 5, the tip 6F for the specimen 6, the tip 6G for the specimen 7, and the tip 6H for the specimen 8. That is, one specimen is specified for each of the eight heads 63A to 63H.

In this example, in the first suction, the tips 6A to 6H suck the cells C of the specimens 1 to 8, respectively. That is, the head unit 61 is moved to all the dishes where the specimens 1 to 8 are supported, and each tip sequentially sucks the cell C of a target specimen in a manner that the tip 6A sucks the cell C of the specimen 1, the tip 6B sucks the cell C of the specimen 2, and so on. The head unit 61 is moved to the microplate 4, and the cells C sucked by the tips 6A to 6H are discharged to the alternate eight wells 41 in the sixteen wells 41 by the first discharging operation. As illustrated in FIG. 10, each one cell C of the specimens 1 to 8 is supported by each of the wells 41 by the first discharge.

Thereafter, similarly, each one cell C of the specimens 1 to 8 is sucked from each dish by each of the eight tips 6A to 6H in the second suction. Thereafter, the head unit 61 is moved to the microplate 4, and the second discharging operation is performed so that the cells C are discharged to the residual eight wells 41 which are not subject to the first discharge from the tips 6A to 6H. As a result, each of the cells C of the specimens 1 to 8 is supported by each two of the sixteen wells 41 for one row. The same operating sequence is performed for necessary rows. When the number of the specimens is large like this example, adopting the method according to the comparative example increases the number of the replacing operations for the tips 6, lengthens the operating time, and increases a number of the tips 6 to be disposed of. However, the present embodiment can shorten the operating time required for transferring cells, and can reduce loss of the tips 6.

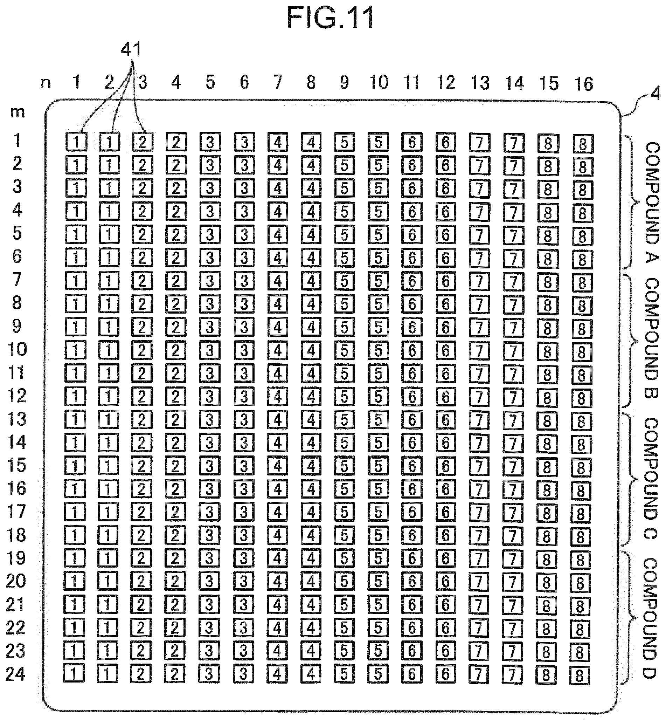

FIG. 11 is a top view illustrating a situation where the cells C are supported in the microplate 4 when the discharging method according to the second embodiment is used. The cells C of the specimen 1 are supported by the wells 41 of m1 to m24 rows × n1 and n2 columns, the cells C of the specimen 2 by the wells 41 of m1 to m24 rows × n3 and n4 columns, the cells C of the specimen 3 by the wells 41 of m1 to m24 rows × n5 and n6 columns, the cells C of the specimen 4 by the wells 41 of m1 to m24 rows × n7 and n8 columns, the cells C of the specimen 5 by the wells 41 of m1 to m24 rows × n9 and n10 columns, the cells C of the specimen 6 by the wells 41 of m1 to m24 rows × n11 and n12 columns, the cells C of the specimen 7 by the wells 41 of m1 to m24 rows × n13 and n14 columns, and the cells C of the specimen 8 by the wells 41 of m1 to m24 rows × n15 and n16 columns.

Like the first embodiment, a "compound A" is put into the wells 41 of m1 to m6 rows × n1 to n16 columns, a "compound B" is put into the wells 41 of m7 to m12 rows × n1 to n16 columns, a "compound C" is put into the wells 41 of m13 to m18 rows × n1 to n16 columns, and a "compound D" is put into the wells 41 of m19 to m24 rows × n1 to n16 columns. After that, the microplate 4 where the cells C are supported can be used for an experiment in which sensitivities of the cells C to the compounds A to D are checked. According to the second embodiment, various processes can be executed simultaneously on eight specimens in one microplate 4.

### [Electrical Configuration of Cell Transfer Apparatus]

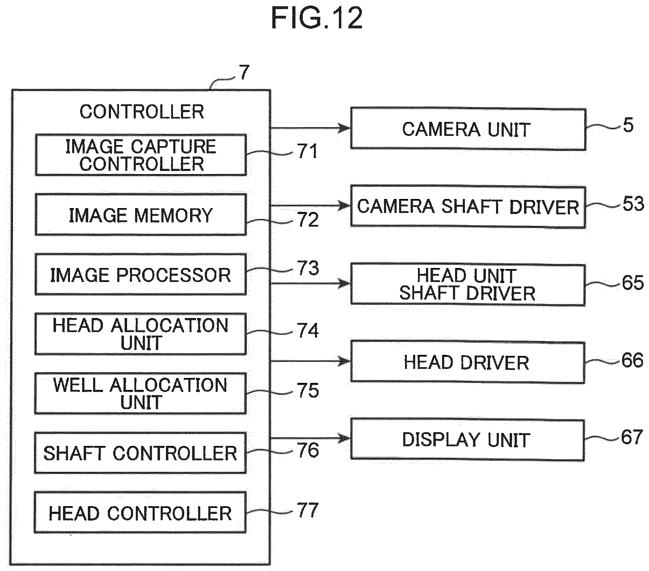

FIG. 12 is a block diagram illustrating an electrical configuration of the cell transfer apparatus S having the above-described functions. The cell transfer apparatus S includes a controller 7 that controls a movement operation of the head unit 61 (FIG. 1), an aligning operation and an up-and-down movement operation of the head 63, an operation for generating a suction force and a discharge force at the head 63 to suck and discharge the cells C, and an operation of the camera unit 5. Further, the cell transfer apparatus S includes a camera shaft driver 53 as a mechanism that horizontally moves the camera unit 5, a head unit shaft driver 65 as a mechanism that horizontally moves the head unit 61, a head driver 66 as a mechanism that causes the head 63 to move up and down and as a mechanism that performs the sucking and discharging operation, and a display unit 67.

The camera shaft driver 53 includes a drive motor that moves the camera unit 5 along the guide rail 52 to any one of the tip imaging position P21, the dish imaging position P22, and the microplate imaging position P23. In a preferable mode, the camera unit 5 that is mounted on a nut member screwed with a ball screw mounted along the guide rail 52 is moved to a target position by the drive motor turning the ball screw in a normal or reverse rotation.

The head unit shaft driver 65 includes a drive motor that moves the head unit 61 (the head main body 62) along the guide rail 64. In a preferable mode, similarly to the camera shaft driver 53, the head unit shaft driver 65 includes a ball screw and a nut member, and the drive motor turns the ball screw in the normal or reverse direction. Note that, when the head main body 62 is moved to X and Y directions, a first ball screw (the X direction) along the guide rail 64, and a second ball screw (the Y direction) mounted on a moving board attached to a first nut member fitted to the first ball screw are used. In this case, the head main body 62 is attached to a second nut member screwed with the second ball screw.

The head driver 66 corresponds to the above-described power unit for the ascending and descending mechanism that moves the head 63 in the up and down direction, and a power unit (for example, a motor) that drives the piston mechanism installed into the hollow portion of the head 63 including a hollow rod. As described above, the ascending and descending mechanism moves the head 63 up and down between a descending position where the head 63 extends downward from the head main body 62 and an ascending position where most part of the head 63 is accommodated in the head main body 62. The power unit of the piston mechanism causes the piston member disposed in the head 63 to move up and down to generate the suction force and the discharge force at the front end opening 6t of the tip 6 attached to the head 63.

The display unit 67 includes, for example, a liquid crystal display, and displays an image captured by the camera unit 5, and an image subject to an image process executed by the controller 7.

The controller 7 includes, for example, a microcomputer, and includes, as functions, an image capture controller 71, an image memory 72, an image processor 73, a head allocation unit 74, a well allocation unit 75, a shaft controller 76, and a head controller 77.

The image capture controller 71 controls an image capturing operation and a movement operation of the camera unit 5. In the present embodiment, the image capture controller 71 causes the camera unit 5 to image the front end openings 6t of the tips 6 attached to the heads 63 in the tip imaging position P21, causes the camera unit 5 to image the dish 2 where the cells C are supported in the dish imaging position P22, and causes the camera unit 5 to image the microplate 4 where the cells C are transferred in the microplate imaging position P23. Note that, in the imaging of the dish 2 or the microplate 4, since the angle of view of the camera unit 5 is considerably small with respect to the dish 2 and the microplate 4, the image capture controller 71 causes the camera shaft driver 53 to slightly move the camera unit 5 in the XY direction and simultaneously causes the camera unit 5 to perform the image capturing operation on the dish 2 or the microplate 4.

The image memory 72 includes, for example, a storage region provided in the microcomputer or an external storage, and temporarily stores image data acquired by the camera unit 5.

The image processor 73 processes the image data captured by the camera unit 5 and stored in the image memory 72. The image processor 73 executes, using an image processing technique, a process for recognizing presence of the cells C on the dish 2 or the microplate 4 through the image, a process for recognizing distribution of the cells C, and a process for recognizing shapes of the recognized cells C, based on, for example, the images of the dish 2 or the microplate 4 where the cells C are held.

When the cells C of a plurality of specimens are present, the head allocation unit 74 determines as to which of the heads 63 is allocated to which of the specimens. For this allocation, the head allocation unit 74 executes a process for specifying each of the heads 63 to be used for transferring each of the specimens. This specification is performed with reference to the number of specimens to be specified by a user, the number of the heads 63 provided to the head main body 62, a suction sequence from a plurality of dishes, and the number of compounds under test. For example, as illustrated in FIG. 8, when the number of specimens is four and the number of the heads 63 (the tips 6) is eight, the head allocation unit 74 specifies the two heads 63 for each of the specimens.

The well allocation unit 75 allocates the wells 41 in the microplate 4 as the specimen wells so that the cells C can be discharged simultaneously from the tips 6 of the specimen heads 63 specified by the head allocation unit 74. For this allocation, the well allocation unit 75 specifies as to which of the wells 41 is used for receiving which of the specimens. For example, when the head specification illustrated in FIG. 8 is set, the well allocation unit 75 allocates 384 wells 41, as illustrated in FIG. 9, so that wells 41 of m1 to m24 rows × n1 to n4 columns (the first specimen wells) are used for the specimen 1, wells 41 of m1 to m24 rows × n5 to n8 columns (the second specimen wells) for the specimen 2, wells 41 of m1 to m24 rows × n9 to n12 columns for the specimen 3, and wells 41 of m1 to m24 rows × n13 to n16 columns for the specimen 4. As a result, the cells C can be transferred to all the wells 41 by performing two simultaneous discharging operations each in which the eight heads 63 are used for one row.

The shaft controller 76 controls an operation of the head unit shaft driver 65. That is, the shaft controller 76 causes the head unit shaft driver 65 to horizontally move the head unit 61 to a predetermined target position. The shaft controller 76 causes the head unit shaft driver 65 to align the heads 63 (the tips 6) with the holding recessed portions 3 of the dish 2 to be subject to a sucking operation over the holding recessed portions 3, to align the heads 63 with the wells 41 in the microplate 4 to be subject to a discharging operation over the wells 41, and to align the tips 6 to be imaged with the image capture pit 17 in the calibrating process.

The head controller 77 controls the head driver 66. The head controller 77 causes the power unit for the ascending and descending mechanism of the head driver 66 to move up and down the heads 63 to be controlled toward a predetermined target position. Further, the head controller 77 controls the power unit of the piston mechanism for the head 63 to be controlled, thus generating a suction force or a discharge force at the front end opening 6t of the tip 6 attached to the head 63 at a predetermined timing.

### [Description of Operation Flow of Cell Transfer Apparatus]

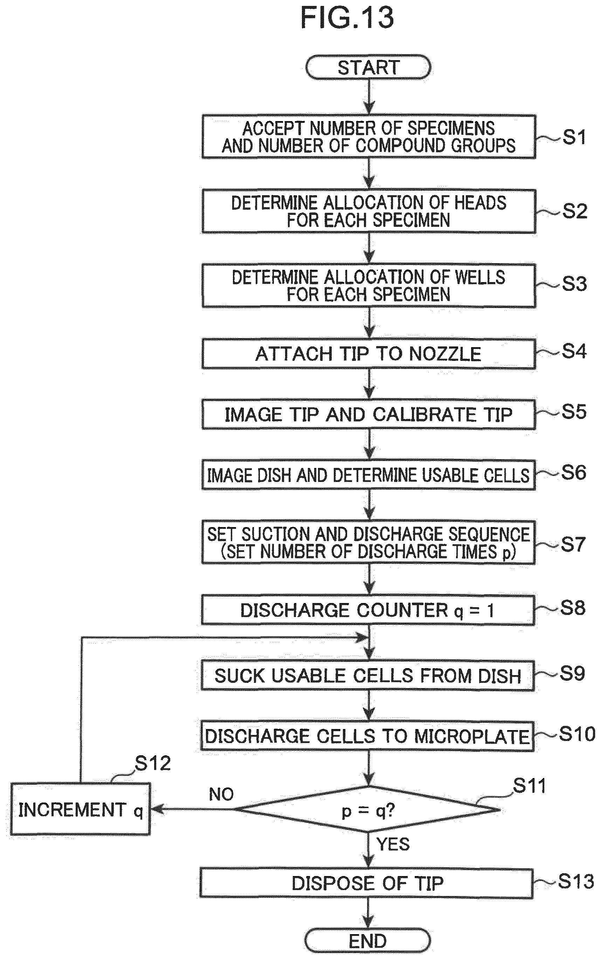

FIG. 13 is a flowchart illustrating an example of an operation of the cell transfer apparatus S. As illustrated in FIG. 1, cell suspensions containing the cells C of the respective specimens are put into the sorting container 18 by the dispensing tips, unillustrated, and the cells C of the specimens are already held on the dish 2. The controller 7 accepts inputs of the number of specimens and the number of compound groups to be used for an experiment through an input device, unillustrated, performed by the user (step S1). Normally, the maximum number Max of specimens is equal to the number of the heads 63 mounted to the head main body 62. Further, the number of specimens is desirably limited to a value that is divisible by the number of the heads 63.

Upon the inputs in step S1, the head allocation unit 74 specifies as to which of the heads 63 (the tips 6) is used for which of the specimens to be subject to the operation for sucking and discharging the cells C (specifies the first and second specimen heads), namely, specifies the heads 63 for the respective specimens (step S2). Its specific examples are illustrated in FIG. 8 and FIG. 10. Thereafter, on the assumption that the cells C are discharged simultaneously from the tips 6 of all the heads 63, the well allocation unit 75 specifies as to which of the tips 6 to be used for discharging the cells C to which of the wells 41 in the microplate 4 (specifies the first and second specimen wells), namely, specifies the wells 41 for the specimens, respectively (step S3). Its specific examples are illustrated in FIG. 9 and FIG. 11.

The above-described steps (1) to (5) are executed. The shaft controller 76 causes the head unit shaft driver 65 to move the head unit 61 to the tip attachment position P11 above the tip stock section 11. At this time, one of unused tips 6 held in the stock container 16 is aligned with the head 63 to which the tip 6 is to be first attached on a vertical axis. Thereafter, the shaft controller 76 causes the head driver 66 to move the aligned head 63 down, and to attach the target tip 6 to a lower end of the head 63 (step S4). In a similar manner, tips 6 are attached to the other heads 63.

The tips 6 are then imaged and calibrated. That is, the shaft controller 76 causes the head unit shaft driver 65 to move the head unit 61 to the tip calibration position P12 above the tip calibrating unit 12. At this time, the head 63 to which the tip 6 is newly attached is aligned with the image capture pit 17 on the vertical axis. Further, the image capture controller 71 causes the camera shaft driver 53 to move the camera unit 5 to the tip imaging position P21 just below the image capture pit 17. Thereafter, the head controller 77 causes the head driver 66 to move down the head 63 to which the tip 6 to be imaged is attached. Further, the image capture controller 71 causes the camera unit 5 to capture an image of the front end opening 6t of the tip 6.

In this image capture operation, while the focus position is being shifted up from a predetermined position below the front end opening 6t as the image capture starting position in tens of microns, the camera unit 5 sequentially captures images of the tip 6. A coordinate position of the front end opening 6t of the tip 6 newly attached to the head 63 is obtained through the contrast detection method illustrated above, for example (step S5). Thereafter, this coordinate position is compared with a reference position, and a correction value is derived from their difference. Similar imaging and similar deriving of the correction value are performed also for the other heads 63.

The image capture controller 71 moves the camera unit 5 to the dish imaging position P22 below the sorting unit 13, and causes the camera unit 5 to capture images of the dish 2 (the dishes 2A to 2D) where the cells C are supported. The acquired image data is stored temporarily in the image memory 72. The image processor 73 executes an image process on the image data to determine usable cells C, and specifies coordinates of the holding recessed portions 3 that support the usable cells C (step S6).

The controller 7 then sets a suction sequence as to in what order the cells C are sucked by the specimen heads 63 (the tips 6) specified by the head allocation unit 74 in step S2. For example, when the cells C of the specimens 1 to 4 are supported by the first to fourth dishes 2A to 2D illustrated in FIG. 2, respectively, the controller 7 determines in what order the sucking operation is performed in the dishes 2A to 2D, or in what order the cells C are sucked from the holding recessed portions 3 in the dishes 2A to 2D respectively, based on the coordinate data obtained in step S6. Further, the controller 7 sets also a discharge sequence as to in what order the cells C are discharged from the heads 63 (the tips 6) to the specimen wells 41 specified by the well allocation unit 75 in step S3 (step S7). The setting of this discharge sequence determines the number of discharge times p at which simultaneous discharge is performed at the tips 6.

Thereafter, the processes for sucking and discharging the usable cells C at the tips 6 are executed. The controller 7 sets a discharge counter q, which indicates the number of simultaneous discharge times at the tips 6, to 1 (step S8). The shaft controller 76 moves the head unit 61 to the cell suction position P13 above the sorting unit 13. At this time, the tips 6 to first perform the sucking operation in the suction sequence are aligned with the holding recessed portions 3 as suction targets in the dish 2 with reference to the correction value obtained in step S5. The head controller 77 causes the heads 63 to move down, generates the suction forces at the heads 63 to suck the cells C from the holding recessed portions 3. Thereafter, the head controller 77 causes the head 63 to move up.

According to the suction sequence, next tips 6 are aligned with next holding recessed portions 3, the heads 63 move down, the cells C are sucked, and the heads 63 move up in a repetitive manner. That is, for example, the cells C of the specimen 1 (the first specimen) are sucked from the first dish 2A by the tips 6 (the first tips) attached to the heads 63 (the first specimen head) specified for the specimen 1, and the cells C of the specimen 2 (the second specimen) are sucked from the second dish 2B by the tips 6 (the second tips) attached to the heads 63 (the second specimen heads) specified for the specimen 2. In such a manner, the cells C of the respective specimens are sequentially sucked (step S9).

The shaft controller 76 moves the head unit 61 to the cell discharge position P14 above the transfer unit 14. At this time, the shaft controller 76 aligns the tips 6 that hold the cells C vertically with the wells 41 as discharge targets in the microplate 4 with reference to the correction value obtained in step S5. Further, the image capture controller 71 moves the camera unit 5 to the microplate imaging position P23 below the transfer unit 14.

The head controller 77 then causes all the heads 63 to move down, generates the discharge force at the heads 63, and causes all the tips 6 to discharge the cells C simultaneously. Thereafter, the head controller 77 causes the head 63 to move up. As a result, the cells C are discharged from the tips 6 (the first tips) that hold the cells C of the specimen 1 to the wells 41 (the first specimen wells) specified for the specimen 1, and the cells C are discharged from the tips 6 (the second tips) that hold the cells C of the specimen 2 to wells 41 (the second specimen wells) specified for the specimen 2. In such a manner, the simultaneous discharging operation is performed (step S10). Note that, in the examples in FIG. 8 and FIG. 10, the two simultaneous discharging operations are performed in every row. Note that the simultaneous discharging operation may be performed in every column. That is, one simultaneous discharging operation or a plurality of simultaneous discharging operations may be performed for wells in one m row or one n column.

After completion of one-cycle sucking and discharging process, the controller 7 checks whether the discharge counter q indicates the set number p of discharge times (step S11). If p is not equal to q (NO in step S11), the controller 7 increments the discharge counter q (step S12), returns to step S9, and executes next sucking and discharging cycle. On the other hand, if p is equal to q (YES in step S11), the controller 7 disposes of the tips 6. Needless to say, when the number of the sucking and discharging cycles is extremely large and contamination of the tips 6 is assumed, before all the sucking and discharging cycles are completed, the controller 7 may execute the process for disposing of the tips 6.

In the disposal process, the shaft controller 76 moves the head unit 61 to the tip disposal position P15 above the tip disposal unit 15. The head controller 77 causes the heads 63 to move down and causes the tip removal rods (unillustrated) contained in the heads 63 to move down, thus pushing the tips 6 out of the heads 63. The pushed-out tips 6 are collected into the tip collecting container 19 (step S13). The controller 7 ends the process for transferring the cells C to one microplate 4.

In the cell transfer apparatus S according to the present embodiment described above, the head allocation unit 74 specifies the plurality of heads 63 as the heads for respective specimens, and thus only the cells C of a specified specimen are allowed to be sucked by or discharged from the tips 6 attached to the heads 63, respectively. That is, for example, the tips 6 that access to the first dish 2A for suction of the cells C of the specimen 1 do not access to the second dish 2B. Therefore, even when the sucking and discharging process is executed multiple times on the cells C by the head unit 61, the tips 6 do not have to be replaced during the process. Accordingly, a time required for the replacing operations for the tips 6 can be omitted, and the number of the tips 6 to be disposed of can be reduced. Further, since the well allocation unit 75 specifies the wells 41 in the microplate 4 so that the cells of the specimens can be discharged simultaneously to the specimen wells, the operation for discharging the cells C can be performed efficiently.

Note that, in the present invention, the cells of the specimens do not necessarily have to be discharged simultaneously to the specimen wells. For example, the discharging operation may be performed in such a manner that the cells C are discharged from the heads 63 specified for transferring the specimen 1 to the wells 41 specified for receiving the specimen 1, and then the cells C are discharged from the heads 63 specified for transferring the specimen 2 to the wells 41 specified for receiving the specimen 2.

Note that the above-described specific embodiments mainly include the invention having the following configurations.

One aspect of the present invention provides a cell transfer apparatus including a dish group including a first dish where a cell of a first specimen is held and a second dish where a cell of a second specimen is held, the first dish and the second dish each having a plurality of holding portions that holds cells to be transferred, a microplate having a plurality of wells that receives the cells, a head unit that is movable between the dish group and the microplate, the head unit including a plurality of heads and tips attached to the heads, respectively, a suction force and a discharge force being generated at the plurality of heads, the tips being configured to suck and discharge the cells, and a controller that controls the generation of the suction force and the discharge force at the plurality of heads and controls movement of the head unit, wherein the controller executes a process for specifying at least some of the plurality of heads as a first specimen head to be used for transferring the first specimen and as a second specimen head to be used for transferring the second specimen, a process for specifying some of the plurality of wells as a first specimen well to be used for receiving the first specimen and as a second specimen well to be used for receiving the second specimen, a process for sequentially sucking the cell of the first specimen from the first dish by the first tip attached to the first specimen head and then the cell of the second specimen from the second dish by the second tip attached to the second specimen head, and a process for discharging the cell of the first specimen from the first tip to the first specimen well and the cell of the second specimen from the second tip to the second specimen well.

In the cell transfer apparatus, since at least some of the plurality of heads are specified as the first specimen head and the second specimen head, the fist tip and the second tip attached to the heads can suck and discharge only the cells of the first specimen and the second specimen, respectively. That is, for example, the first tip that accesses to the first dish for sucking the cell of the first specimen does not access to the second dish. For this reason, even when the sucking and discharging process is executed multiple times on the cells through the head unit, the tips do not have to be replaced during the process. Accordingly, a time required for the replacing operations for the tips can be omitted, and the number of the tips to be disposed of can be reduced.

In the cell transfer apparatus, it is desirable that the controller specifies the first specimen well and the second specimen well so that simultaneous discharge is performable at the first tip attached to the first specimen head and at the second tip attached to the second specimen head, and simultaneously discharges the cell of the first specimen at the first tip and the cell of the second specimen at the second tip in the discharging process.

The cell transfer apparatus can perform the cell discharging operation efficiently because the wells of the microplate are specified so that the cells of the first specimen and the second specimen can be discharged simultaneously to the first and second specimen wells, respectively.

In the cell transfer apparatus, it is preferable that the plurality of wells in the microplate is arranged into m row × n column, and the plurality of heads are arranged in one line with an arrangement pitch that is p-multiple, p being an integer of 1 or more, of an arrangement pitch of the wells on the m row or n column.

This cell transfer apparatus can perform the operation for discharging cells simultaneously to a plurality of wells more efficiently.

Desirably, the cell transfer apparatus further includes a tip stock section where the tips that are unused are stored, and a tip calibrating unit that obtains positions of front end openings of the tips attached to the plurality of heads, wherein before the sucking process, the controller executes control of moving the head unit to the tip stock section and attaching the tips that are unused to the plurality of heads, and control of moving the head unit to the tip calibrating unit and obtaining positions of the front end openings of the tips newly attached to the plurality of heads.

In this cell transfer apparatus, unused tips are attached to the heads in the tip stock section, and the tip calibrating unit obtains positions of the front end openings of the tips. According to the present invention, in the cell transfer apparatus having such a function, new tips are attached to the heads and the number of operations for obtaining the positions of the front end openings of the new tips can reduced.

It is desirable that the cell transfer apparatus further includes a tip disposal unit that collects the tips that has been used from the plurality of heads.

This cell transfer apparatus includes the function for collecting tips from the heads, and can reduce the number of tip disposal operations.

According to the above-described present invention, there is provided the cell transfer apparatus, which transfers the cells to the microplate having wells for receiving the cells from a dish in which the cells are held, can transfer a plurality of types of cells to the microplate efficiently and can reduce the number of tips to be disposed of.

## Claims

1. A cell transfer apparatus comprising:
a dish group including a first dish where a cell of a first specimen is held and a second dish where a cell of a second specimen is held, the first dish and the second dish each having a plurality of holding portions that holds cells to be transferred;
a microplate having a plurality of wells that receives the cells;
a head unit that is movable between the dish group and the microplate, the head unit including a plurality of heads and tips attached to the heads, respectively, a suction force and a discharge force being generated at the plurality of heads, the tips being configured to suck and discharge the cells; and
a controller that controls the generation of the suction force and the discharge force at the plurality of heads and controls movement of the head unit,
wherein the controller executes
a process for specifying at least some of the plurality of heads as a first specimen head to be used for transferring the first specimen and as a second specimen head to be used for transferring the second specimen,
a process for specifying some of the plurality of wells as a first specimen well to be used for receiving the first specimen and as a second specimen well to be used for receiving the second specimen,
a process for sequentially sucking the cell of the first specimen from the first dish by the first tip attached to the first specimen head and then the cell of the second specimen from the second dish by the second tip attached to the second specimen head, and
a process for discharging the cell of the first specimen from the first tip to the first specimen well and the cell of the second specimen from the second tip to the second specimen well.

2. The cell transfer apparatus according to claim 1, wherein the controller specifies the first specimen well and the second specimen well so that simultaneous discharge is performable at the first tip attached to the first specimen head and at the second tip attached to the second specimen head, and simultaneously discharges the cell of the first specimen at the first tip and the cell of the second specimen at the second tip in the discharging process.

3. The cell transfer apparatus according to claim 2,
wherein the plurality of wells in the microplate is arranged into m row × n column, and
wherein the plurality of heads are arranged in one line with an arrangement pitch that is p-multiple, p being an integer of 1 or more, of an arrangement pitch of the wells on the m row or n column.

4. The cell transfer apparatus according to any one of claims 1 to 3, further comprising:
a tip stock section where the tips that are unused are stored; and
a tip calibrating unit that obtains positions of front end openings of the tips attached to the plurality of heads,
wherein before the sucking process, the controller executes control of moving the head unit to the tip stock section and attaching the tips that are unused to the plurality of heads, and control of moving the head unit to the tip calibrating unit and obtaining positions of the front end openings of the tips newly attached to the plurality of heads.

5. The cell transfer apparatus according to any one of claims 1 to 4, further comprising a tip disposal unit that collects the tips that has been used from the plurality of heads.
